# EUROPEAN PATENT APPLICATION

(11) **EP 0 968 691 A2**
(43) Date of publication of application: **05.01.2000**
(21) Application number: 99304220.9
(22) Date of filing: 28.05.1999
(51) Int. Cl.: A61F 2/30

(54) **Prosthesis provided with a mantle**

(30) Priority: 04.06.1998 GB 9812069
(71) Applicant: BENOIST GIRARD ET CIE, 14201 Hérouville-Saint-Clair Cedex (FR)
(72) Inventor: Ling, Robin Sydney Mackwood, Prof., Dartmouth, Devon TQ6 OHB (GB); Gie, Graham Allan, Yeoford, Devon EX17 5EZ (GB); Timperley, Andrew John, Exeter, EX2 4PA (GB); Storer, John Andrew, 14250 Jouaye Mondaye, Bayeux (FR)
(74) Representative: Bridge-Butler, Alan James

(57) **Abstract**

A prosthesis (1) provided with a stem (2) at least part of which is enclosed in a mantle (3) and which is for insertion in a bone cavity (4) and attachment thereto by cement (5), said mantle (3) having a wall thickness which is increased towards its proximal end (6) to provide an outwardly projecting proximal balcony (8) and which is dimensioned and adapted to locate the stem (2) in a predetermined position within the cross-sectional area of the bone cavity (4) at or towards the proximal end (7) thereof.

## Description

This invention relates to a prosthesis provided with a mantle and a mantle for use with such a prosthesis.

It is known to provide the stem of a femoral prosthesis with an outer mantle, for example as shown in EP 0 457 464.

The object of the present invention is to provide a mantle which not only performs the usual function of preventing contact between metal and bone but can also assist in pressurising the cement.

According to the present invention in a prosthesis provided with a stem at least part of which is enclosed in a mantle and which is for insertion in a bone cavity and attachment thereto by cement, said mantle having a wall thickness which is increased towards its proximal end to provide an outwardly projecting proximal balcony and which is dimensioned and adapted to locate the stem in a predetermined position within the cross-sectional area of the bone cavity at or towards the proximal end thereof.

With this construction the proximal surfaces beneath the balcony can act to provide a compression on the cement as the prosthesis provided with the mantle is inserted into the bone cavity.

The outer shape of the mantle can be formed with one or more concave surfaces the proximal end or ends of which form said balcony.

Alternatively the outer shape of the mantle can be formed with one or more substantially flat surfaces the proximal ends of which form said balcony.

In one preferred construction the mantle has an outer shape which is conical or frusto-conical and thus it has only one outer surface which provides the concave surface referred to above.

In another construction the mantle can have an outer shape which includes three or more flat surfaces which are curved inwardly from its proximal end. With such a construction the mantle can have a cross-sectional outer shape which is triangular, square or rectangular.

The mantle can be arranged to cover the stem from its distal tip or adjacent thereto to a point adjacent the proximal cut end of the bone when the stem is inserted.

With this arrangement the distal end of the mantle can enclose the stem and be provided with a cup into which the distal end of the stem can subsequently sink and said cup can be arranged to extend up to half the length of the stem from its distal end.

If desired the mantle can be provided with means for distally centralising the stem in the bone canal.

In another construction the mantle can be arranged to cover the stem from a point adjacent the proximal end of the bone when the stem is inserted to a point spaced away from the distal tip.

In a convenient construction the mantle is preformed and subsequently fitted to the stem.

The mantle can be made from any convenient material, for example polymethylmethacrylate (PMMA).

The invention also includes a preformed mantle for use with a prosthesis provided with a stem and which is for insertion in a bone cavity and attachment thereto by bone cement, said mantle being shaped to engage the outer surface of the stem and having a wall thickness which is increased towards its proximal end to provide an outwardly projecting proximal balcony.

This mantle can have all the features referred to above, for example, the outer shape of the mantle can be formed with one or more concave surfaces, the proximal end or ends of which form said balcony or it could have one or more substantially flat surfaces as referred to above.

The invention can be performed in many ways and some embodiments will now be described by way of example and with reference to the accompanying drawings in which :
Figure 1 is an isometric view of part of a mantle according to the invention;
Figure 2 is a cross-sectional side elevation of a femoral prosthesis provided with a mantle according to the invention;
Figures 3 and 4 are views similar to Figure 2 showing different forms of mantle;
Figure 5 is a view similar to Figure 2 showing another form of mantle;
Figure 6 is a cross-sectional side elevation showing the shortened form of mantle in position on a femoral prosthesis; and,
Figure 7 is a cross-sectional side elevation of another form of mantle according to the invention.

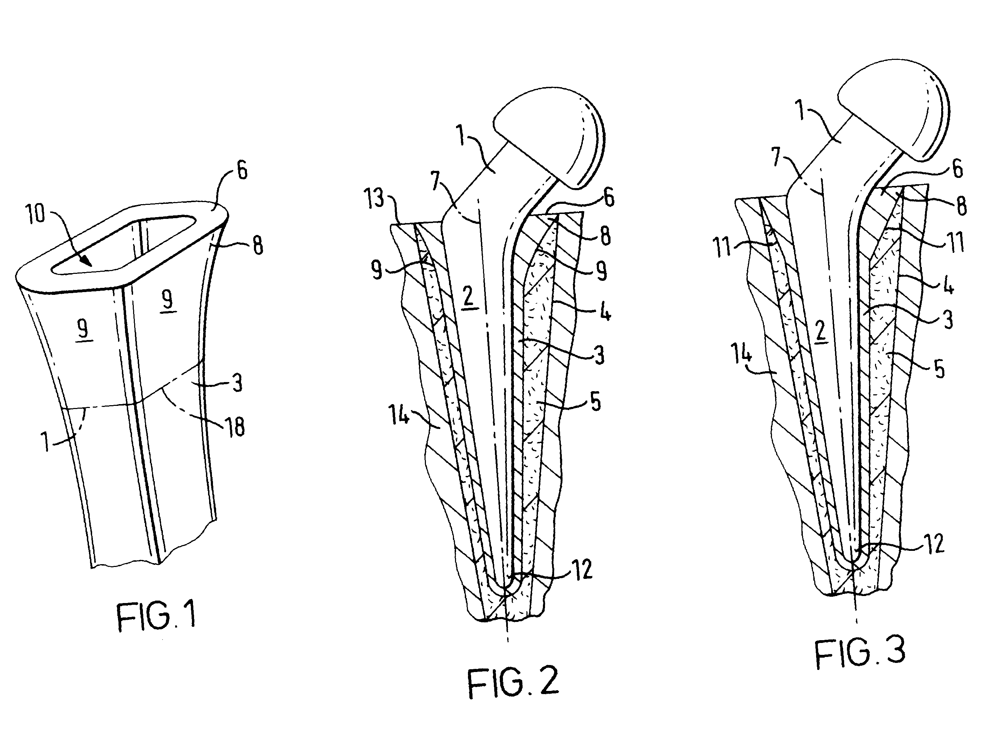

As shown in Figures 1 and 2 a prosthesis 1 according to the invention is provided with a stem 2 which is enclosed in a mantle 3 and which is shown inserted in a bone cavity 4 filled with cement 5. As will be seen from Figure 2 the wall thickness of the mantle 3 is increased towards its proximal end 6 which is adjacent the proximal end 7 of the stem 2 to provide an outwardly projecting balcony 8. Concave surfaces 9 are provided, the proximal ends of which form the balcony 8.

In the construction shown in Figures 1 and 2 the mantle is used on a stem having flat sides and thus the proximal end opening 10 is substantially rectangular as will be seen from Figure 1. The mantle has an outer shape which includes four flat concave surfaces 9 and which are curved inwardly from the proximal end 6.

The mantle covers the stem from its distal tip 12 to a point adjacent the proximal cut end 13 of the bone 14 when the stem is inserted.

The distal end of the mantle need not necessarily cover the distal end of the stem 2.

It will be seen that the proximal end of the balcony 8 is shaped to engage the walls 4 of the bone cavity so that when the cavity has been filled with cement 5 and the prosthesis is inserted into it the concave surfaces 9 act to pressurise the cement as the final part of the entry is made. As the balcony engages the side walls 4 of the cavity it acts to guide the prosthesis and at the same time seal the cavity and prevent escape of cement.

Another construction according to the invention is shown in Figure 3 in which the same reference numerals are used to indicate the same parts as those shown in Figures 1 and 2. In this construction the concave surfaces 4 are replaced by substantially flat surfaces 11 the proximal ends of which form the balcony 8. The effect is similar to that described with regard to Figures 1 and 2.

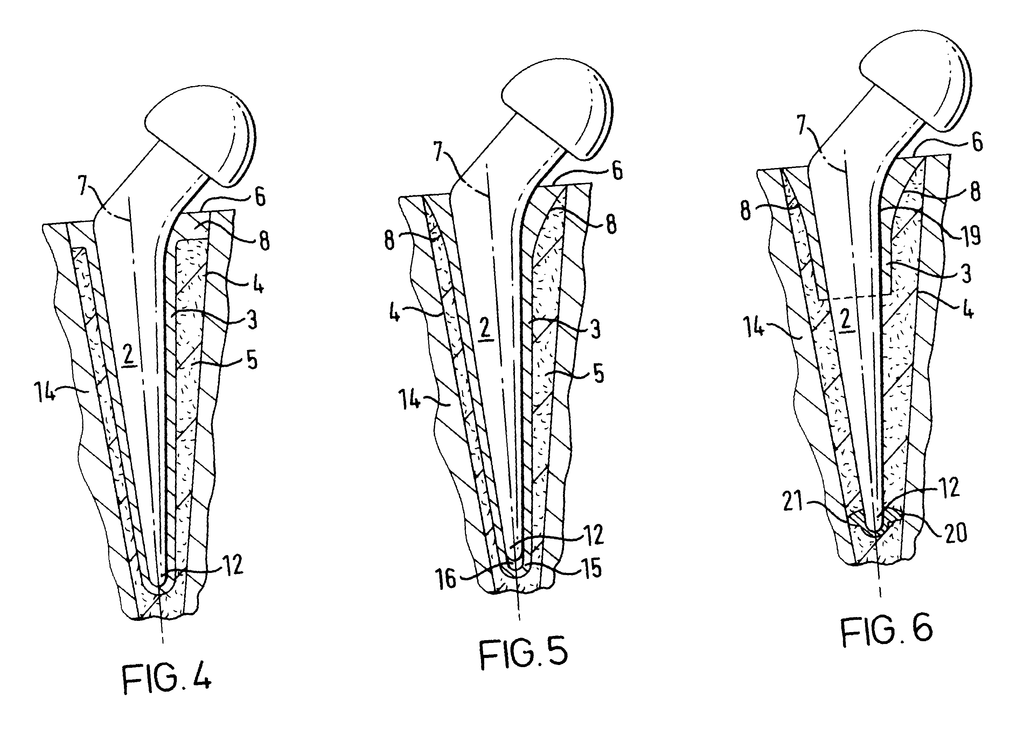

Figure 4 shows another alternative construction in which the same reference numerals as those used in Figures 1 and 2 are again employed for similar parts. In the construction the surface of the balcony 8 is cut back to provide what is, in effect, a flange which once again operates in the manner described.

The mantle can be provided with a distal centraliser, for example of the kind shown in EP 0 457 464 or a separate centraliser could be employed with is applied externally of the mantle.

Figure 5 shows another construction similar to that shown in Figures 1 and 2 and the same reference numerals are used to indicate similar parts. In this construction however the distal end 15 of the mantle is extended to provide a void 16 into which the stem 2 can subsequently sink after insertion. The balcony shape could be as shown in any of Figures 1 to 4.

The mantle can be formed on the stem of the prosthesis or it can be preformed as a separate article and suitably dimensioned and adapted for subsequent fitting to the stem of the prosthesis.

The mantle can be made from any suitable material but is preferably made from a material similar to bone cement, for example polymethylmethacrylate (PMMA) so that it has a good adherence with the bone cement used in the bone cavity.

In the arrangements described above the cross-section of the mantle is substantially rectangular but surfaces beneath the balcony could be triangular, square or any other convenient shape which is suitable for the particular cross-sectional shape of the prosthesis stem.

Again, if the prosthesis stem is circular in cross-section then flat or concave surfaces can again be used or the outer shape of the mantle can be substantially conical or frusto-conical beneath the balcony 8 thus providing a single surface at that portion.

Figure 1 also shows an alternative form of mantle which can be used as shown in Figure 6. Once again similar reference numerals are used to indicate similar parts. In this construction the mantle 3 can be a similar shape to that shown in the other constructions but the mantle only extends downwardly for the distance shown above the broken lines 18 in Figure 1. As will be seen from Figure 6 the mantle only extends for about one third of the length of the stem and is indicated by reference numeral 19. This mantle acts in a similar way to that described with regard to the other Figures and again acts as a proximal guide for the stem 2. An additional distal centraliser 20 is shown which is located on the distal end 12 of the stem 2 and is provided with radially extending location wings or fins 21. The stabiliser can be made from any convenient material and is preferably resilient so that it can locate in the bone cavity appropriately.

As described with regard to Figures 2 and 3 the mantle 19 can be made as a separate article and be subsequently fitted to the stem.

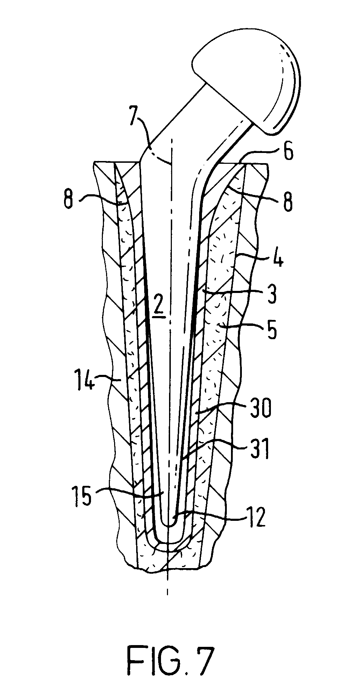

Figure 7 shows another construction similar to that shown in Figure 5 and the same reference numerals are used to indicate similar parts. In this construction however the mantle is shaped and dimensioned to only engage the proximal part of the stem down to about half way or less. The distal portion 30 of the mantle can be parallel sided or tapered so that it creates a void 31 which extends up to half of the length of the mantle from the distal end. This acts to limit the length of engagement of the stem with the mantle, the intention being to limit the load transfer between the stem and the bone so that it takes place in the proximal portion of the bone opening.

This concept can also be applied to the construction shown in Figures 1, 2, 3 or 4. It will again be appreciated that the mantle can be preformed as a separate article and suitably dimensioned and adapted for subsequent fitting to the stem of the prosthesis.

In all the constructions described above the outer edges or surfaces of the sleeve are shown in contact with the wall of the bone cavity 4. In certain circumstances this may be undesirable and the sleeve can therefore be dimensioned so that there is a gap around the contact surfaces which will be filled with cement. The gap will be small enough to ensure that the guiding effect of the sleeve is achieved but intimate contact of the sleeve material with the inner wall of the bone cavity is avoided. Thus, there will always be some cement dough which then polymerises between the preformed mantle and the endosteal surface of the femur. This will ensure that the cement can take a "mirror image" cast of the inside of the relevant part of the canal and such a cast may be important for the subsequent formation of living bone in contact with the cement. The overall effect however is similar to that which is achieved if the sleeve makes actual contact with the inner wall.

## Claims

1. A prosthesis provided with a stem at least part of which is enclosed in a mantle and which is for insertion in a bone cavity and attachment thereto by cement, said mantle having a wall thickness which is increased towards its proximal end to provide an outwardly projecting proximal balcony and which is dimensioned and adapted to locate the stem in a predetermined position within the cross-sectional area of the bone cavity at or towards the proximal end thereof.

2. A prosthesis as claimed in claim 1 in which the mantle is formed with one or more concave surfaces, the proximal end or ends of which form said balcony.

3. A prosthesis as claimed in claim 1 in which the outer shape of the mantle is formed with one or more substantially flat surfaces the proximal ends of which form said balcony.

4. A prosthesis as claimed in claim 1 in which said mantle has an outer shape which includes three or more flat surfaces which are curved inwardly from its proximal end.

5. A prosthesis as claimed in claim 4 in which said mantle has a cross-sectional shape which is triangular, square or rectangular.

6. A prosthesis as claimed in claim 1 or claim 2 in which said mantle has an outer shape which is conical or frusto-conical.

7. A prosthesis as claimed in any one of the preceding claims 1 - 6 in which said mantle covers the stem from its distal tip or adjacent thereto to a point adjacent the proximal cut end of the bone when inserted.

8. A prosthesis as claimed in any one of claims 1 to 7 in which the distal end of the mantle encloses said stem and is provided with a cup into which the distal end of said stem can subsequently sink.

9. A prosthesis as claimed in claim 8 in which said cup is arranged to extend up to half the length of the stem from its distal end.

10. A prosthesis as claimed in any one of claims 1 to 9 in which said mantle is provided with means for distally centralising the stem in the bone canal.

11. A prosthesis as claimed in any one of preceding claims 1 to 6 in which said mantle covers said stem from a point adjacent the proximal end of the bone when the stem is inserted to a point spaced away from the distal tip.

12. A prosthesis as claimed in any one of preceding claims in which said mantle is preformed and subsequently fitted to the stem.

13. A prosthesis as claimed in any one of preceding claims in which said mantle is made from a synthetic material.

14. A prosthesis as claimed in claim 13 in which said mantle is made from polymethylmethacrylate (PMMA).

15. A preformed mantle for use with a prosthesis provided with a stem and which is for insertion in a bone cavity and attachment thereto by bone cement, said mantle being shaped to engage the outer surface of the stem and having a wall thickness which is increased towards its proximal end to provide an outwardly projecting proximal balcony.

16. A preformed mantle as claimed in claim 15 which is formed with one or more concave surfaces, the proximal end or ends of which form said balcony.

17. A preformed mantle as claimed in claim 15 in which the outer shape is formed with one or more substantially flat surfaces the proximal ends of which form said balcony.

18. A preformed mantle as claimed in claim 15 which has an outer shape which includes three or more flat surfaces which are curved inwardly from its proximal end.

19. A preformed mantle as claimed in claim 18 which has a cross-sectional shape which is triangular, square or rectangular.

20. A preformed mantle as claimed in claim 15 or claim 16 which has an outer shape which is conical or frusto-conical.

21. A preformed mantle as claimed in any one of the preceding claims 15 - 20 which is adapted to cover the stem of the prosthesis with which it is to be used from the distal tip thereof or adjacent thereto to a point adjacent the proximal cut end of the bone when inserted.

22. A preformed mantle as claimed in any one of claims 15 to 21 in which the distal end thereof is adapted to enclose the stem of the prosthesis with which it is to be used and is provided with a cup into which the distal end of said stem can subsequently sink.

23. A preformed mantle as claimed in claim 22 in which said cup is arranged to extend up to half the length of the stem from its distal end.

24. A preformed mantle as claimed in any one of claims 15 to 23 which is provided with means for distally centralising the stem of the prostheses with which it is to be used in a bone canal.

25. A preformed mantle as claimed in any one of preceding claims 15 to 20 which is adapted to cover said stem from a point adjacent the proximal end of the bone when the stem is inserted to a point spaced away from the distal tip thereof.

26. A preformed mantle as claimed in any one of preceding claims which is preformed and adapted to be subsequently fitted to the stem with which it is to be used.

27. A preformed mantle as claimed in any one of preceding claims 15 to 16 which is made from a synthetic material.

28. A preformed mantle as claimed in claim 27 which is made from polymethylmethacrylate (PMMA).
